# EUROPÄISCHE PATENTSCHRIFT

(11) **EP 1 779 887 B1**
(45) Veröffentlichungstag und Bekanntmachung des Hinweises auf die Patenterteilung: **04.05.2011**
(21) Anmeldenummer: 06090186.5
(22) Anmeldetag: 12.10.2006
(51) Int. Cl.: A61M 16/08

(54) **Schlauchset**
Tube set
Système de tubes

(30) Priorität: 28.10.2005 DE 102005052122
(43) Veröffentlichungstag der Anmeldung: 02.05.2007
(73) Patentinhaber: W.O.M. World of Medicine AG, 10553 Berlin (DE)
(72) Erfinder: Heath, Andy, 10553 Berlin (DE)
(74) Vertreter: Jungblut, Bernhard Jakob

(56) Entgegenhaltungen:
- EP-A- 0 839 551
- EP-A- 1 576 971
- WO-A-2005/028012
- DE-U1- 9 320 713
- US-A1- 2005 038 374

## Beschreibung

### Gebiet der Erfindung

Die Erfindung betrifft ein Schlauchset für eine Vorrichtung zum Insufflieren von Gas in einen menschlichen oder tierischen Körper mit einem ersten Anschluss, welcher an die Vorrichtung zum Insufflieren anschließbar ist, mit einem weiteren Anschluss, welcher an ein Insufflationsinstrument anschließbar ist, wobei das Schlauchset ein Sterilfilterelement enthält, welches den Innenquerschnitt überspannt. Die Erfindung betrifft des Weiteren eine Vorrichtung zum Insufflieren von Gas mit einem solchen Schlauchset.

### Stand der Technik und Hintergrund der Erfindung

Schlauchsets der eingangs genannten Art werden insbesondere im Bereich der Endoskopie bzw. Laparoskopie verwendet. Die typischerweise eingesetzte Gasart ist Kohlendioxyd, wobei selbstverständlich auch andere Gase, ggf. mit pharmazeutischen Zusatzstoffen, verwendet werden können. Als Insufflationsinstrument kommt beispielsweise eine Veress-Nadel in Frage.

Aus der Literaturstelle DE 3922746 C1 ist es bekannt, ei-nen Sterilfilter zwischen Insufflationsinstrument und Gasversorgungseinrichtung einzusetzen. Der Sterilfilter wird dabei in den Schlauch, welcher zwischen der Gasversorgungseinrichtung und dem Insufflationsinstrument eingerichtet ist, eingefügt. Im Rahmen der insofern bekannten Maßnahmen kann nicht mit hinreichender Sicherheit ausgeschlossen werden, dass eine Bedienperson gar keinen Sterilfilter oder einen gebrauchten Sterilfilter einsetzt mit der Folge einer gesundheitlichen Gefährdung des Patienten.

Aus der Literaturstelle EP 0684850 B1 ist ebenfalls ein Filterelement bekannt, welches zwischen Schlauchleitung und Gasquelle eingesetzt wird. Es handelt sich auch hier um ein übliches, separat zu handhabendes und beidseitig anzuschließendes Filterelement.

Aus der Literaturstelle DE 19955847 C1 ist eine Vorrichtung zum Insufflieren von Gas bekannt, wobei ein Sterilfilterelement in das anzuschließende Schlauchset integriert ist und einen Teil des Anschlusses des Schlauchsets an der Gasversorgungseinrichtung darstellt.

Insbesondere ein Schlauchset gemäß der Literaturstelle DE 19955847 C1 hat sich grundsätzlich sehr gut bewährt. Im Lichte des zunehmenden Kostendrucks im Gesundheitswesen ist es jedoch wünschenswert, zumindest Teile eines Schlauchsets mehrfach nutzen zu können.

Aus dem Dokument US 2005/0038374 ist ein Kit, enthaltend ein Schlauchset mit einem Filterelement bekannt, welches zur Insufflation und Entnahme von Körperflüssigkeiten, insbesondere zur Entnahme von Stuhlproben dient.

### Technisches Problem der Erfindung

Der Erfindung liegt daher das technische Problem zugrunde, ein Schlauchset für eine Vorrichtung zum Insufflieren von Gas anzugeben, welches zumindest teilweise wiederverwendbar ist, und zwar bei gleichzeitig erhaltener sehr hoher Sicherheit in Hinblick auf die Sterilität.

Grundzüge der Erfindung und bevorzugte Ausführungsformen

Zur Lösung dieses technischen Problems lehrt die Erfindung ein Schlauchset für eine Vorrichtung zum Insufflieren von Gas in einen menschlichen oder tierischen Körper, mit einem ersten Schlauchteil, welcher einen ersten Anschluss und einen zweiten Anschluss aufweist, wobei der erste Anschluss an die Vorrichtung zum Insufflieren anschließbar ist, mit einem zweiten Schlauchteil, welcher einen dritten Anschluss und einen vierten Anschluss aufweist, wobei der vierte Anschluss an ein Insufflationsinstrument anschließbar ist, wobei der zweite Anschluss ein erstes Verbindungselement und der dritte Anschluss ein zweites Verbindungselement aufweisen, wobei das erste Verbindungselement und das zweite Verbindungselement mechanisch zueinander komplementär ausgebildet sind, wobei mittels der Verbindungselemente der zweite Anschluss und der dritte Anschluss gasdicht miteinander verbunden sind, und wobei der dritte Anschluss ein Sterilfilterelement enthält, welches den Innenquerschnitt des dritten Anschlusses überspannt und wobei das erste Verbindungselement im Bereich des Umfangs seines Flansches einen ersten elektrischen Anschluss trägt und das zweite Verbindungselement im Bereich des Umfangs seines Flansches einen zweiten elektrischen Anschluss trägt, wobei mit dem zweiten Anschluss eine im zweiten Schlauchteil angeordnete Heizvorrichtung elektrisch verbunden ist und wobei entweder das erste Verbindungselement im Bereich des Umfangs seines Flansches eine elektrische Leseeinheit trägt und das zweite Verbindungselement im Bereich des Umfangs seines Flansches ein maschinenlesbares Identelement trägt, wobei das Identelement und die Leseeinheit in der Endstellung der beiden Flansche einander unmittelbar gegenüber stehen oder eine Gasversorgungseinrichtung, an welche das Schlauchset angeschlossen ist, eine elektrische Leseeinheit trägt und das zweite Verbindungselement im Bereich des Umfangs seines Flansches ein maschinenlesbares ldentelement in Form eines Transponders trägt.

Hierbei ist wesentlich, dass jedenfalls der zweite Anschluss ein integrales Bauteil des ersten Schlauchteils und der dritte Anschluss ein integrales Bauteil des zweiten Schlauchteils ist. Dies bedeutet, dass die jeweiligen Schläuche von den betreffenden Anschlüssen im normalen Einsatz nicht lösbar sind. Es versteht sich, dass die beiden Verbindungselemente gasdicht miteinander verbindbar sind, ggf. unter Einsatz einer Dichtung.

Mit der Erfindung wird ein Schlauchset geschaffen, welches im Kern zweiteilig ist. Das zweite Schlauchteil mit dem Sterilfilterelement ist dabei zum Austausch nach jeder Benutzung bestimmt. Das erste Schlauchteil kann dagegen mehrfach verwendet und beispielsweise nur alle 24 Stunden ausgetauscht werden. Somit muss nicht das gesamte Schlauchset nach jeder Benutzung erneuert werden, sondern lediglich das zweite Schlauchteil, wodurch eine erhebliche Kostenersparnis erreicht wird. Zudem wird der Anfall an entsorgungsbedürftigen Abfällen beachtlich reduziert.

Vorzugsweise trägt das zweite Verbindungselement das Sterilfilterelement, i.e. das Sterilfilterelement ist hierein fest eingebaut. Dabei kann sich das Sterilfilterelement über den gesamten Innenquerschnitt des zweiten Verbindungselementes erstrecken. Es ist aber auch möglich, dass sich das Sterilfilterelement nur über einen Teil des Innenquerschnitts erstreckt, wobei dann der Rest des Innenquerschnitts durch eine gasdichte Wandung abgeschlossen ist.

Im Einzelnen können die zueinander komplementären Verbindungselemente in verschiedenen Varianten ausgebildet sein.

Zum Ersten ist es möglich, dass eines der Verbindungselemente durch einen Flansch mit einem Außengewinde und das andere Verbindungselement durch einen Flansch mit einem zum Außengewinde komplementären Innengewinde gebildet ist, wobei die Flansche durch Verschrauben miteinander in eine Endstellung gasdicht verbunden werden.

Zum Zweiten kann es eingerichtet sein, dass eines der Verbindungselemente durch einen Flansch mit von einander be-abstandeten und radial nach innen ragenden Rastvorsprüngen und das andere Verbindungselement durch einen Flansch mit sich in radialer Richtung erstreckenden Haltevorsprüngen gebildet ist, wobei die Haltevorsprünge, bezogen auf die Umfangsrichtung des Flansches, einen Abstand aufweisen, welcher größer als die Länge der Rastvorsprünge ist, und wobei die Haltevorsprünge und die Rastvorsprünge durch Verdrehen der beiden Flansche gegeneinander in eine Endstellung miteinander in Eingriff bringbar sind und die Flansche gasdicht miteinander verbinden. Eine solche Ausbildung der Verbindungselemente wird auch als Bajonettverschluss bezeichnet.

Zum Dritten ist es möglich, dass eines der Verbindungselemente durch einen Flansch mit zumindest einer im Bereich des Umfangs des Flansches angeordneten Rastausnehmung und das andere Verbindungselement durch einen Flansch mit zumindest einem im Bereich des Umfangs angeordneten Rastelement gebildet sind, wobei Rastausnehmung und Rastelement zueinander komplementär sind und durch Einrasten ineinander in eine Endstellung die Flansche gasdicht miteinander verbinden. In dieser Ausführungsform wird es sich empfehlen, mehrere Rastausnehmungen und Rastelemente komplementär zueinander vorzusehen und um den Umfang der Flansche zu verteilen. Die Anzahl der Rastelemente bzw. Rastausnehmungen kann hierbei im Bereich von zwei bis zehn, vorzugsweise drei bis fünf, liegen. In einer besonderen Ausfürhungsform dieser Variante trägt eines der Verbindungselemente eine Flanschhülse, in welche der Flansch des anderen Verbindungselementes einsteckbar ist. Die Flanschhülse trägt dabei innenseitig einen umlaufenden Rastvorsprung und der Flansch um den Außenumfang eine umlaufende Rastausnehmung. Beim Ineinanderstecken der Bauteile rasten Rastvorsprung und Rastausnehmung gasdicht ineinander ein. Selbstverständlich kann stattdessen die Flanschhülse eine innenseitig umlaufende Rastausnehmung aufweisen und der Flansch einen um den Ausßenumfang umlaufenden Rastvorsprung. Rastausnehmungen und/oder Rastelement bzw. Rastvorsprung können aus einem elastischen oder plastischen Werkstoff gebildet sein, beispielsweise einem üblichen Kunststoff.

Schließlich kann in einer weiteren Variante eines der Verbindungselemente eine Flanschhülse aufweisen, deren Innendurchmesser im Öffnungsbereich größer als der Außendurchmesser des Flansches des anderen Verbindungselements und wobei der Innendurchmesser der Flanschhülse, bezogen auf die Einschubrichtung des Flansches des anderen Verbindungselementes, abnimmt auf einen Innendurchmesser, welcher geringer als der Außendurchmesser des Flansches des anderen Verbindungselementes ist, wobei zumindest eines der Bauteile, Flanschhülse und/oder Flansch aus einem plastischen oder elastischen Werkstoff gebildet sind. Eine kinematische Umkehrung des Vorstehenden ist eingerichtet, wenn eines der Verbindungselemente eine Flanschhülse mit zylinderförmiger Innenwandung aufweist, wobei der Außendurchmesser des Flansches, bezogen auf die Einschubrichtung des Flansches des anderen Verbindungselementes, abnimmt von einem Außendurchmesser, welcher größer als der Innendurchmesser der Flanschhülse ist, auf einen Außendurchmesser, welcher kleiner als als der Innendurchmesser der Flanschhülse ist. Selbstverständlich ist es auch möglich, dass sowohl Innendurchmesser der Flanschhülse und Außendurchmesser des Flansches im vorstehenden Sinne konisch ausgebildet sind. Die Abnahme des Innendurchmessers bzw. Außendurchmessers kann dabei kontinuierlich sein. Beim festen Einstecken des Flansches in die Flanschhülse entsteht dann in jeder dieser varianten ein Kraftschluss, welcher die beiden Verbindungselemente zusammen hält. Als Werkstoffe kommen grundsätzlich alle üblichen Kunststoffe in Frage, da diese die erforderliche Elastizität bzw. Plastizität aufweisen. Es wird sich jedoch empfehlen, keine Kunststoffe mit sehr niedrigen Reibwerten, wie beispielsweise Teflon, einzusetzen, damit der Kraftschluss durch Reibung hinreichend für eine sichere Verbindung ist. Die Konizität der Innenseite der Flanschhülse bzw. des Außendurchmessers des Flansches kann im Bereich von 0,1 bis 5°, vorzugsweise 0,1 bis 2°, bezogen auf die Mittenachse der Flanschhülse, betragen.

Den vorstehenden Varianten der Verbindungselemente ist gemeinsam, dass die Flansche im Zuge des Verbindens in axialer Richtung ineinander bzw. zueinander bewegt werden. Hierdurch können ringförmige Dichtflächen in den Verbindungselementen gegeneinander in Kontakt gebracht werden, wodurch die Gasdichtigkeit der Verbindung gegen die Umgebung gewährleistet ist. Zu diesem Zwecke kann in zumindest einem der verbindungselemente eine ringförmige Dichtung, beispielsweise eine aus einem Elastomer, vorgesehen sein.

Die feste Verbindung des Sterilfilterelements im zweiten Schlauchteil gewährleistet, dass mit einem Verwerfen des Sterilfilterelements auch gleichzeitig der hiermit verbundene zweite Schlauchteil verworfen wird und umgekehrt. Auch ist ein Gebrauch des Schlauchsets ohne Sterilfilterelement nicht möglich.

Als Sterilfilterelement kann beispielsweise als Membranfilter mit einer Durchflussleistung von 5 bis 100 1/min, vorzugsweise von 16 bis 60 1/min, ausgebildet sein. Als Material kommt beispielsweise PTFE mit einer Porengröße von 1 bis 5 µm, vorzugsweise ca. 3 µm, in Frage. Solche Membranfilter haben eine Filtrationseffizienz von 99,9998 %. Grundsätzlich sind jedoch auch alle anderen, eine hinreichende Keimretention bewirkende Filterelementarten mit ausreichend niedrigem Gasströmungswiderstand einsetzbar.

Bei dem ersten Anschluss und dem vierten Anschluss kann es sich um handelsübliche Steck-, Schraub- oder Bajonettanschlüsse handeln, wobei entsprechende komplementäre Anschlüsse in der Gasversorgungseinrichtung bzw. dem Insüfflationsinstrument vorgesehen sind.

In einer besonders bevorzugten Ausführungsform weist entweder das erste Verbindungselement im Bereich des Umfangs seines Flansches oder eine Gasversorgungeinrichting, an welche das Schlauchset angeschlossen ist, eine elektrische Leseeinheit auf, und das zweite verbindungselement trägt im Bereich des Umfangs seines Flansches ein maschinenlesbares Identelement, wobei das Identelement und die Leseeinheit in der Endstellung der beiden Flansche einander unmittelbar gegenüber stehen. Bei miteinander verbundenem ersten Verbindungselement und zweiten Verbindungselement bzw. bei an die Gasversorgungseinrichtung angeschlossenem Schlauchset kann folglich die Leseeinheit das Identelement auslesen. Als Identelement wird ein Merkmal bezeichnet, welches Informationen bezüglich des Sterilfilterelements enthält. Maschinenlesbare Identelemente sind in den verschiedensten Ausführungsformen bekannt und im Rahmen der Erfindung einsetzbar. Beispiele hierfür sind: Barcode, Magnetstreifen, Mikrochip und Hologramm. Im Falle des Magnetstreifens sowie des Microchips ist es auch möglich, den Informationsgehalt des Identelements zu verändern und/oder zu ergänzen, wobei dann die Leseeinheit zusätzlich in geeigneter Weise als Schreibeinheit ausgebildet sein muss oder eine zusätzliche Schreibeinheit aufweist. Im Falle des Barcodes ist die Leseeinheit ein optischer Scanner. Im Falle des Magnetstreifens ist die Leseeinheit ein Magnetlesekopf, welcher ggf. auch gleichzeitig als Schreibkopf genutzt werden kann. Im Falle des Mikrochips ist die Leseeinheit ein Kontaktfeld, welches zugeordnete Kontaktfelder des Identelements bei verbundenen Verbindungselementen kontaktiert. Im Falle des Hologramms umfasst die Leseeinheit eine Quelle kohärenten Lichts sowie eine geeignete optische Sensorik. Bei den vorstehend genannten konkreten Varianten wird die leseeinheit in der Regel am ersten Verbindungselement vorgesehen sein. Des Weiteren kann das Identelement ein Transponder sein. Dann ist die Leseeinheit eine Transponderabfrageeinrichtung. In dieser Variante kann die Leseeinheit nicht nur am ersten Verbindungselement, sondern alternativ oder zusätzlich auch an der Gasversorgungseinrichtung vorgesehen sein, da je nach eingesetzter transpondertechnologie eine Auslesung auch über eine Distanz von bis zu 0,5 m, sogar bis zu 1,0 m und mehr, möglich ist. In jedem Fall findet eine geeignete Umwandlung der von der Leseeinheit aufgenommenen Eigenschaften bzw. Strahlungen in elektrische Signale statt, welche ggf. nach A/D-Wandlung einer vorzugsweise digitalen Auswerteeinheit als Informationssignale zugeführt werden. In der Auswerteeinheit wird die mittels der Leseeinheit aus dem Identelement ausgelesene Information ausgewertet und nach Maßgabe des Ergebnisses dieser Auswertung die Gasversorgungseinrichtung freigeschaltet oder gesperrt. Hierzu umfasst die Auswerteeinheit Speicherelemente, in welchen die Information sowie die Referenzinformation abspeicherbar bzw. permanent abgespeichert sind. In dieser Ausführungsform lässt sich eine absichtliche oder missbräuchliche Wiederverwendung bereits gebrauchter zweiter Schlauchteile zuverlässig durch geeignete Maßnahmen im Rahmen der Auswerteeinheit verhindern. So ist es beispielsweise möglich, dass das Identelement ein für jedes zweite Schlauchteil verschiedenes Codeelement (beispielsweise eine fortlaufende Nummerierung codierend) aufweist. Bei der Erstverwendung eines zweiten Schlauchteils wird dann das Codeelement eingelesen und im Rahmen der Auswerteeinheit als "aktuell" gespeichert und anhand entsprechend zuvor als "verbraucht" gespeicherte Codeelemente durch Vergleich überprüft, ob das aktuelle individuelle Codeelement zuvor bereits einmal eingelesen wurde. Im Falle des tatsächlichen Ersteinsatzes ist dies nicht der Fall und die Gasversorgungseinrichtung wird freigeschaltet. Die Information des aktuellen Codeelements wird dann (zum Beispiel nach Entfernung des zweiten Schlauteils) als "verbraucht" abgespeichert. Wenn dagegen das individuelle Codeelement bereits vorher einmal eingelesen wurde, i.e. bereits als "verbraucht" abgespeichert ist, so handelt es sich nicht mehr um eine Erstverwendung des betreffenden zweiten Schlauchteils, und die Auswerteeinheit sperrt dann die Gasversorgungseinrichtung und gibt ggf. akustische oder optische Signale. Wird z.B. mit einem Magnetstreifen, einem Mikrochip und/oder einem Transponder gearbeitet, so kann im Rahmen einer Erstbenutzung alternativ oder zusätzlich ein Sperrcode in das Identelement geschrieben werden. Wenn dann eine Wiederverwendung des zweiten Schlauchteils versucht wird, so identifiziert die Auswerteeinheit den mittels der Leseeinheit eingelesenen Sperrcode mit der bereits zuvor geschilderten Folge eines Alarmsignals und/oder einer Sperrung der Gasversorgungseinrichtung. In dieser Ausführungsform ist sichergestellt, dass auch eine Wiederverwendung eines zweiten Schlauchteils an verschiedenen Geräten nicht durchgeführt werden kann.

Insgesamt wird mit der Ausführungsform mit einem maschinenlesbaren Identelement eine sehr hohe Betriebssicherkeit des erfindungsgemäßen Schlauchsets gewährleistet. Denn es bleibt nicht mehr einer Bedienperson überlassen, ob ein bereits benutztes zweites Schlauchteil nochmals verwendet werden könnte. Ein ordnungsgemäßer Betrieb ist nämlich nur dann möglich, wenn ein neues, i.e. ungebrauchtes, zweites Schlauchteil angeschlossen worden ist.

In einer vorteilhaften Weiterbildung dieser Variante umfasst das Identelement ein Sicherheitselement. Ein Sicherheitselement meint ein manuell, visuell oder maschinenlesbares Element, welches mit einer hohen Fälschungssicherheit ausgestattet ist. Sicherheitselemente, auch maschinenlesbare Sicherheitselemente, sind beispielsweise aus der Technologie der Wertpapiere und Zahlungsmittel mannigfaltig bekannt und können in entsprechender Weise im Rahmen der vorliegenden Erfindung eingesetzt werden. Ein maschinenlesbares Sicherheitselement ist beispielsweise durch ein Hologramm gebildet. Hologramme lassen sich nur unter vergleichsweise sehr hohem technologischen Aufwand nachahmen, so dass eine Anbringung von gefälschten Hologrammen im Rahmen der Erfindung wirtschaftlich unattraktiv wird. Im Falle von maschinenlesbaren Sicherheitselementen (neben Hologrammen kommen hierfür beispielsweise auch magnetische Sicherheitselemente usw. in Frage) kann eine automatische Sperre der Gasversorgungseinrichtung bei Fehlen des Sicherheitselements und/oder den Anforderungen nicht genügendem Sicherheitselement durch die Auswerteeinheit bewirkt werden, wenn die Leseeinheit zum Lesen des Sicherheitselements eingerichtet ist und/oder eine Sicherheitselement-Leseeinrichtung zusätzlich vorgesehen ist. Mit einem solchen Sicherheitselement wird eine beachtlich erhöhte Sicherheit für Patienten erreicht, da zweite Schlauchteile von nicht autorisierten Herstellern, insbesondere von mit minderer Qualität arbeitenden Billiganbietern, von der Auswerteeinheit nicht akzeptiert werden. Eine Bedienperson kann folglich ausschließlich zweite Schlauchteile von den qualitätsüberwachten autorisierten Herstellern ansetzen, um die Gasversorgungsvorrichtung in Betrieb zu nehmen. Ein Sicherheitselement kann auch im Rahmen des Identelements als verschlüsseltes Sicherheitscodeelement eingerichtet sein.

Das erste Verbindungselement kann im Bereich des Umfangs seines Flansches einen ersten elektrischen Anschluss und das zweite Verbindungselement im Bereich des Umfangs seines Flansches einen zweiten elektrischen Anschluss tragen, wobei mit dem zweiten Anschluss ein im zweiten Schlauchteil und abströmungsseitig des Sterilfilterelements angeordneter Feuchtigkeitssensor eingerichtet ist. In der Endstellung der Verbindungselemente sind die beiden elektrischen Anschlüsse miteinander kontaktiert. Des Weiteren kann im zweiten Schlauchteil eine mit dem zweiten Anschluss elektrisch verbundene Heizvorrichtung vorgesehen sein.

Über eine Drahtverbindung vom ersten elektrischen Anschluss zur Vorrichtung zum Insufflieren kann der Feuchtigkeitssensor ausgelesen bzw. die Heizvorrichtung mit Strom versorgt werden.

Mit dem ersten elektrischen Anschluss kann unabhängig vom Vorstehenden eine Drahtverbindung zum Anschluss an die Vorrichtung zum Insufflieren verbunden sein, über welche die aus dem Identelement ausgelesene Information in die Auswerteeinheit übermittelt wird. Alternativ kann dies aber auch drahtlos erfolgen. So ist es möglich, dass mit dem ersten elektrischen Anschluss ein Sender zur drahtlosen Übertragung von Daten zur Vorrichtung zum Insufflieren elektrisch verbunden ist. Dies kann ein Funksender oder ein IR-Sender sein.

In der Ausführungsform mit Flüssigkeitssensor ist Folgendes ergänzend zu erläutern. Zwar sind bzw. können Filtereinheiten hydrophob und insofern flüssigkeitssperrend ausgebildet sein. Diese hydrophoben Materialeigenschaften sind allerdings von den Herstellern nur über bestimmte Zeiträume garantiert. Aus Sicherkeitsgründen ist es daher empfehlenswert, wenn eine mit Flüssigkeit kontaktierte Filtereinheit verworfen wird. In der hier beschriebenen Ausführungsform dient der Flüssigkeitssensor der Detektion eines Vordringens von Flüssigkeit bzw. eines Aerosols zur Filtereinheit. Daher empfiehlt es sich, dass der Feuchtigkeitssensor nahe der Filtereinheit angebracht ist. Wenn der Feuchtigkeitssensor über den zweiten elektrischen Anschluss und den ersten elektrischen Anschluss die Überschreitung eines vorgegebenen Grenzwertes meldet, so wird in der Auswerteeinheit ein Alarmsignal ausgelöst und/oder die Gasversorgungseinrichtung angehalten. Weiterhin ist es möglich, dass das zweite Schlauchteil dann automatisch als "verbraucht" gekennzeichnet wird, beispielsweise durch Sperrung des Codeelements in der Auswerteeinheit und/oder Schreiben eines Sperrcodes in das Identelement.

Des Weiteren betrifft die Erfindung eine Vorrichtung zum Insufflieren von Gas in einen menschlichen oder tierischen Körper mit einer Gasversorgungseinrichtung mit einem Gasanschluss, an welchem ein erfindungsgemäßes Schlauchset mittels des ersten Anschlusses angeschlossen ist, und mit einem an dem vierten Anschluss angeschlossenen Insufflationsgerät. Je nach Ausführungsform des erfindungsgemäßen Schlauchsets kann die Gasversorgungseinrichtung mit einem elektrischen Anschluss versehen sein, an welchen die Leseeinheit und/ oder der erste elektrische Anschluss des Schlauchsets lösbar anschließbar ist. Es kann auch vorgesehen sein, dass im Rahmen der Vorrichtung ein Empfänger zum Empfang von mittels des Senders des Schlauchsets übertragenen Daten eingerichtet ist.

Eine Gasversorgungseinrichtung weist typischerweise einen Anschluss an eine Gasquelle, beispielsweise eine Kohlendioxydquelle, einen Druckminderer, einen Durchflussmesser, einen Druckmesser sowie eine Steuerschaltung/Auswerteeinheit zur Druck- und/oder Durchflusssteuerung auf. Es versteht sich, dass die genannten Bauelemente in geeigneter Weise über Gasleitungen (Schläuche oder Rohre) miteinander verbunden sind. Mittels der Steuerschaltung wird über geeignete Stellglieder Druck und Durchfluss einstellt. Hierzu ist die Steuerschaltung mit dem Durchflussmesser und dem Druckmesser verbunden. Im Rahmen der Erfindung ist an die Steuerschaltung des Weiteren die Leseeinheit anschließbar. An die Steuerschaltung kann des Weiteren der Feuchtigkeitssensor angeschlossen sein. Schließlich kann die Steuerschaltung eine eingerichtete Heizvorrichtung elektrisch ansteuern.

Im Folgenden wird die Erfindung anhand von lediglich eine Ausführungsform darstellenden Figuren näher erläutert. Es zeigen:
- Figur 1:: eine schematische Übersichtsdarstellung einer erfingungsgemäßen Vorrichtung zum Insufflieren von Gas,
- Figur 2:: eine Detailansicht eines erfindungsgemäßen Schlauchsets im Bereich der Verbindungselemente in der Ausführungsform mit Gewinde,
- Figur 3:: Ansichten in axialer Richtung erfindungsgemäßer Verbindungselemente mit Bajonettverschluss und
- Figur 4:: eine Detailansicht zweier Verbindungselemente mit einer Rastverbindung.

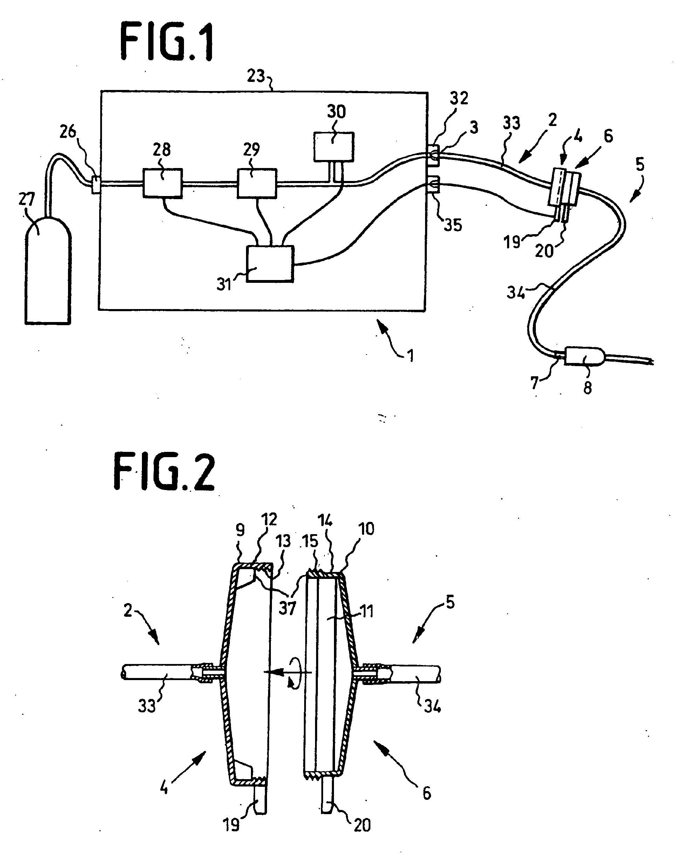

In der Figur 1 erkennt man eine Vorrichtung 1 zum Insufflieren von Gas für die Laparoskopie. Als Insufflationsinstrument 8 kommt eine Veress-Nadel zum Einsatz, welche im Zuge eines ärztlichen Eingriffes in einen menschlichen Körper eingeführt wird. Die Gasversorgungseinrichtung 23 umfasst einen Anschluss 26 an eine Kohlendioxydquelle 27, einen Druckminderer 28, einen Durchflussmesser 29, einen Druckmesser 30 sowie eine Steuerschaltung und Auswerteeinheit 31. Das Schlauchset ist mittels eines ersten Anschlusses 3 mit einem Gasauslass 32 der Gasversorgungseinrichtung 23 verbunden.

Des Weiteren erkennt man ein erfindungsgemäßes Schlauchset mit einem ersten Schlauchteil 2, welcher einen ersten Anschluss 3 und einen zweiten Anschluss 4 aufweist. Der erste Anschluss 3 und der zweite Anschluss 4 sind über einen ersten Schlauch 33 miteinander verbunden. Des Weiteren ist ein zweites Schlauchteil 5 eingerichtet, welches einen dritten Anschluss 6 und einen vierten Anschluss 7 aufweist, wobei der vierte Anschluss 7 an ein Insufflationsinstrument 8 anschließbar ist. Des Weiteren erkennt man, dass im Rahmen des zweiten Anschlusses 4 eine Leseeinheit 19 eingerichtet ist, welche über eine Drahtverbindung und ein Kontaktpaar 35 mit der Auswerteeinheit 31 verbunden ist. Der Leseeinheit 19 unmittelbar gegenüber steht ein Identelement 20.

Der Schlauch 33 ist mit dem zweiten Anschluss 4 fest verbunden. Desgleichen ist der Schlauch 34 mit dem dritten Anschluss 6 fest verbunden. Fest verbunden meint hierbei, dass der jeweilige Schlauch von dem jeweiligen Anschluss nicht ohne weiteres manuell entfernbar ist. Dies bedeutet auch, dass der zweite Anschluss und der dritte Anschluss keine separaten baulichen Einheiten darstellen, auf welche ein Schlauch 33, 34 manuell aufsteckbar ist.

In der Figur 2 ist eine Detailansicht in Bereich des zweiten Anschlusses und des dritten Anschlusses einer ersten Ausführungsform der Erfindung dargestellt. Der zweite Anschluss 4 weist ein erstes verbindungselement 9 auf. Der dritte Anschluss 6 weist ein zweites Verbindungselement 10 auf. Im Rahmen des dritten Anschlusses 6 ist ein Sterilfilterelement 11 eingerichtet, welches den Innenquerschnitt des dritten Anschlusses 6 überspannt. In der Ausführungsform der Figur 2 ist das Verbindungselement 9 durch einen Flansch 12 mit einem Gewinde 13 gebildet. Das Verbindungselement 10 weist ebenfalls einen Flansch 14 auf, welcher jedoch mit einem zum Innengewinde 13 komplementären Außengewinde 15 ausgestattet ist. Die Verbindungselemente 9, 10 werden durch Ineinanderdrehen miteinander verbunden. Hierbei kommen Dichtflächen 35 gegeneinander zum Anliegen, wodurch in der Endstellung eine gasdichte Verbindung eingerichtet ist. Zwischen den Dichtflächen 35 kann auch eine elastomere Ringdichtung eingesetzt werden. In der Endstellung stehen sich die Leseeinheit 19 und das Identelement 20 unmittelbar gegenüber. Dies bedeutet, dass die beiden Verbindungselemente 9 und 10 gegen die Kraft einer (elastomeren) Dichtfläche 35 bzw. der Ringdichtung so weit ineinander gedreht werden, bis die Leseeinheit 19 und das Identelement 20 einander gegenüberstehen, wodurch auch eine stets definierte Montage der beiden Verbindungselemente 9, 10 und zuverlässige Abdichtung gewährleistet ist. Hierbei kann an der Leseeinheit 19 oder dem Identelement 20 auch ein Anschlagselement vorgesehen sein, dessen Höhe größer als der Abstand zwischen Leseeinheit 19 und Identelement 20 in der Endstellung ist, jedoch kleiner als dieser Abstand zuzüglich der Höhe eines Gewindegangs.

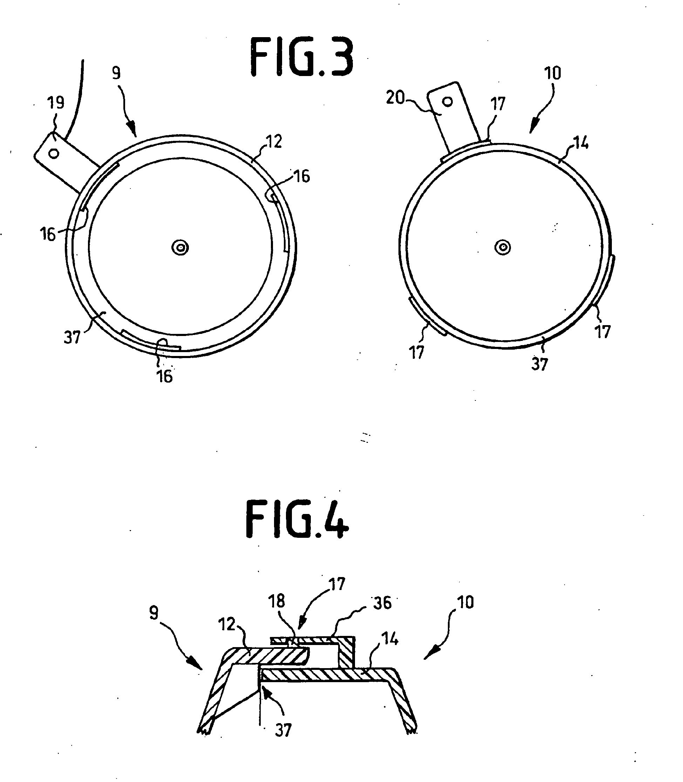

In der Ausführungsform der Figur 3 ist anstelle des Außengewindes 15 und des Innengewindes 13 ein sog. Bajonettverschluss eingerichtet. Hierzu weist das Verbindungselement 9 einen Flansch 12 mit voneinander beabstandeten und radial nach innen ragenden Rastvorsprüngen 16 auf. Das Verbindungselement 10 weist ebenfalls einen Flansch 14 auf, welcher jedoch mit sich in radialer Richtung erstreckenden Haltevorsprüngen 17 ausgestattet ist. Ebenso wie in der vorstehenden Ausführungsform versteht es sich, dass der Flansch 14 einen Außendurchmesser aufweist, welcher gleich oder geringfügig kleiner als der Innendurchmesser des Flansches 12 ist. Wird nun das Verbindungselement 10 so in das Verbindungselement 9 eingeführt, dass die Haltevorsprünge 17 zwischen den Rastvorsprüngen 16 angeordnet sind, so kann das Verbindungselement 10 gegen das Verbindungselement 9 so verdreht werden, dass die Haltevorsprünge 17 die Rastvorsprünge 16 hintergreifen. Hierbei wird es sich empfehlen, wenn die Rastvorsprünge 16 und/oder die Haltevorsprünge 17 in der Umfangsrichtung und Drehrichtung axial schräg verlaufen mit der Maßgabe, dass das Verbindungselement 10 im Zuge des Eindrehens in das Verbindungselement 9 in dieses in axialer Richtung hineingezogen wird. Hierdurch kommen die Dichtflächen 35 gegeneinander abdichtend in Kontakt. Nicht dargestellt ist, dass im Rahmen der Rastvorsprünge 16 und/ oder der Haltevorsprünge 17 Endanschlagselemente vorgesehen sein können. Hierbei werden die Endanschlagselemente mit der Maßgabe angeordnet sein, dass bei Anliegen die Leseeinheit 19 und das Identelement 20 einander unmittelbar gegenüberliegen. In der dargestellten Ausführungsform wird es sich empfehlen, dass die Rastvorsprünge 16 sowie die Haltevorsprünge 17 nicht gleichmäßig über den Umfang verteilt, sondern vielmehr nicht-rotationssymmetrisch angeordnet sind. Hierdurch wird gewährleistet, dass der Bajonettverschluss nur in einer einzigen definierten Stellung der Verbindungselemente 9 und 10 zueinander in Eingriff gebracht werden kann.

In der Figur 4 erkennt man eine weitere Variante der Erfindung, wobei das Verbindungselement 10 im Bereich des Umfangs des Flansches 14 mit einer Rastausnehmung 17 ausgestattet ist. Das Verbindungselement 9 weist im Bereich des Umfangs des Flansches 12 ein Rastelement 18 auf. Rastausnehmung 17 und Rastelement 18 sind zueinander komplementär ausgebildet. Die Rastausnehmung 17 ist auf einem elastisch biegbaren Rastarm 36 angeordnet.

In den Figuren der Übersichtlichkeit halber nicht dargestellt ist, dass das zweite Schlauchteil 5 einen Feuchtigkeitssensor und optional eine Heizvorrichtung aufweist. Beide sind über im Rahmen der Leseeinheit 19 und dem Identelement 20 eingerichtete Kontaktpaare kontaktiert und mit der Steuer- und Auswerteeinheit 31 verbunden.

## Patentansprüche

1. Schlauchset für eine Vorrichtung (1) zum Insufflieren von Gas in einen menschlichen oder tierischen Körper im Rahmen der Laparoskopie,
mit einem ersten Schlauchteil (2), welcher einen ersten Anschluss (3) und einen zweiten Anschluss (4) aufweist, wobei der erste Anschluss an die Vorrichtung (1) zum Insufflieren anschließbar ist, und
mit einem zweiten Schlauchteil (5), welcher einen dritten Anschluss (6) und einen vierten Anschluss (7) aufweist, wobei der vierte Anschluss (7) an ein Insufflationsinstrument (8) anschließbar ist,
wobei der zweite Anschluss (4) ein erstes Verbindungselement (9) und der dritte Anschluss (6) ein zweites Verbindungselement (10) aufweisen, wobei das erste Verbindungselement (9) und das zweite Verbindungselement (10) mechanisch zueinander komplementär ausgebildet sind, wobei mittels der Verbindungselemente (9, 10) der zweite Anschluss (4) und der dritte Anschluss (6) gasdicht miteinander verbindbar sind, und
wobei der zweite oder der dritte Anschluss (6) ein Sterilfilterelement (11) enthält, welches den Innenquerschnitt des dritten Anschlusses (6) überspannt,
**dadurch gekennzeichnet, dass**
wobei das erste Verbindungselement (9) im Bereich des Umfangs seines Flansches (12) einen ersten elektrischen Anschluss (21) trägt und das zweite Verbindungselement (10) im Bereich des Umfangs seines Flansches (14) einen zweiten elektrischen Anschluss (22) trägt, wobei mit dem zweiten Anschluss (22) eine im zweiten Schlauchteil (5) angeordnete Heizvorrichtung elektrisch verbunden ist
weiterhin **dadurch gekennzeichnet, dass** entweder
das erste Verbindungselement (9) im Bereich des Umfangs seines Flansches (12), eine elektrische Leseeinheit (19) trägt und das zweite Verbindungselement (10) im Bereich des Umfangs seines Flansches (14) ein maschinenlesbares Identelement (20) trägt, wobei das Identelement (20) und die Leseeinheit (21) in der Endstellung der beiden Flansche (12, 14) einander unmittelbar gegenüber stehen
oder
eine Gasversorgungseinrichtung (23), an welche das Schlauchset angeschlossen ist, eine elektrische Leseeinheit (19) trägt und das zweite Verbindungselement (10) im Bereich des Umfangs seines Flansches (14) ein maschinenlesbares Identelement (20) in Form eines Transponders trägt.

2. Schlauchset nach Anspruch 1, wobei das zweite Verbindungselement (10) das Sterilfilterelement (11) trägt.

3. Schlauchset nach Anspruch 1 oder 2, wobei eines der Verbindungselemente (9, 10) durch einen Flansch (12) mit einem Innengewinde (13) und das andere Verbindungselement (9, 10) durch einen Flansch (14) mit einem zum Innengewinde (13) komplementären Außengewinde (15) gebildet ist und wobei die Flansche (12, 14) durch Verschrauben miteinander in eine Endstellung gasdicht miteinander verbunden werden.

4. Schlauchset nach Anspruch 1 oder 2, wobei eines der Verbindungselemente (9, 10) durch einen Flansch (12) mit voneinander beabstandeten und radial nach innen ragenden Rastvorsprüngen (16) und das andere Verbindungselement (9, 10) durch einen Flansch (14) mit sich in radialer Richtung erstreckenden Haltevorsprüngen (17) gebildet ist, wobei die Haltevorsprünge (17), bezogen auf die Umfangsrichtung des Flansches (14), einen Abstand aufweisen, welcher größer als die Länge der Rastvorsprünge (16) ist, und wobei die Haltevorsprünge (17) und die Rastvorsprünge (16) durch Verdrehen der beiden Flansche (12, 14) gegeneinander in eine Endstellung miteinander in Eingriff bringbar sind und die Flansche (12, 14) gasdicht miteinander verbinden.

5. Schlauchset nach Anspruch 1 oder 2, wobei eines der Verbindungselemente (9, 10) durch einen Flansch (14) mit zumindest einer im Bereich des Umfangs des Flansches (14) angeordneter Rastausnehmung (17) und das andere Verbindungselement (9, 10) durch einen Flansch (12) mit zumindest einem im Bereich des Umfangs angeordneten Rastelement (18) gebildet sind, wobei Rastausnehmung (17) und Rastelement (18) zueinander komplementär sind und durch Einrasten ineinander in eine Endstellung die Flansche (12, 14) gasdicht miteinander verbinden.

6. Schlauchset nach Anspruch 2 oder 3,
wobei eines der Verbindungselemente (9, 10) eine Flanschhülse aufweist, deren Innendurchmesser im Öffnungsbereich größer als der Außendurchmesser des Flansches (14) des anderen Verbindungselements (9, 10) und wobei der Innendurchmesser der Flanschhülse, bezogen auf die Einschubrichtung des Flansches (14) des anderen Verbindungselementes (9, 10), abnimmt auf einen Innendurchmesser, welcher geringer als der Außendurchmesser des Flansches (14) des anderen Verbindungselementes (9, 10) ist, wobei zumindest eines der Bauteile, Flanschhülse und/oder Flansch (14) aus einem plastischen oder elastischen Werkstoff gebildet sind, oder
wobei eines der Verbindungselemente (9, 10) eine Flanschhülse mit zylinderförmiger Innenwandung aufweist, wobei der Außendurchmesser des Flansches (14), bezogen auf die Einschubrichtung des Flansches (14) des anderen Verbindungselementes (9, 10), abnimmt von einem Außendurchmesser, welcher größer als der Innendurchmesser der Flanschhülse ist, auf einen Außendurchmesser, welcher kleiner als als der Innendurchmesser der Flanschhülse ist, wobei zumindest eines der Bauteile, Flanschhülse und/oder Flansch (14) aus einem plastischen oder elastischen Werkstoff gebildet sind.

7. Schlauchset nach einem der Ansprüche 1 bis 6, wobei das erste Verbindungselement (9) im Bereich des Umfangs seines Flansches (12) einen ersten elektrischen Anschluss (21) trägt und das zweite Verbindungselement (10) im Bereich des Umfangs seines Flansches (14) einen zweiten elektrischen Anschluss (22) trägt, wobei mit dem zweiten Anschluss (22) ein im zweiten Schlauchteil (5) angeordneter Feuchtigkeitssensor elektrisch verbunden ist.

8. Schlauchset nach Anspruche 7, wobei mit dem ersten elektrischen Anschluss (21) eine Drahtverbindung zum Anschluss an die Vorrichtung (1) zum Insufflieren und/oder einen Sender zur drahtlosen Übertragung von Daten zur Vorrichtung (1) zum Insufflieren elektrisch verbunden ist.

9. Schlauchset nach Anspruch 1, wobei das Identelement (20) ein für jedes zweite Schlauchteil (5) verschiedenes Codeelement aufweist, und wobei das Identelement (20) vorzugsweise ein Sicherheitselement aufweist.

10. Vorrichtung (1) zum Insufflieren von Gas in einen menschlichen oder tierischen Körper im Rahmen der Laparoskopie mit einer Gasversorgungseinrichtung (23) mit einem Gasanschluss (24), an welchem ein Schlauchset nach einem der Ansprüche 1 bis 10 mittels des ersten Anschlusses (3) angeschlossen ist, und mit einem an den vierten Anschluss (7) angeschlossenen Insufflationsinstrument (8) sowie mit einer Steuerschaltung zur Ansteuerung der Heizvorrichtung des zweiten Schlauchteils (5).

11. Vorrichtung nach Anspruch 10, mit einem elektrischen Anschluss (25), an welchen die Leseeinheit (19) und/ oder der erste elektrische Anschluss (21) des Schlauchsets lösbar anschließbar ist, und/oder mit einem Empfänger zum Empfang von mittels des Senders des Schlauchsets übertragener Daten.

## Claims

1. A tube set for a device (1) for insufflating gas into a human or animal body during laparoscopy,
with a first tube portion (2) that comprises a first connection (3) and a second connection (4), the first connection being connectable to the device (1) for insufflating, and
with a second tube portion (5) that comprises a third connection (6) and a fourth connection (7), the fourth connection (7) being connectable to an insufflation instrument (8),
the second connection (4) comprising a first connecting element (9), and the third connection (6) comprising a second connecting element (10), the first connecting element (9) and the second connecting element (10) being configured mechanically complementary to each other, by means of the connecting elements (9, 10) the second connection (4) and the third connection (6) being connectable with each other in a gas-tight manner, and
the second or the third connection (6) including a sterile filter element (11) that spans the inner cross section of the third connection (6),
**characterized in that**
the first connecting element (9) carries in the region of the periphery of its flange (12) a first electrical connection (21), and the second connecting element (10) carries in the region of the periphery of its flange (14) a second electrical connection (22), with the second connection (22) a heating device arranged in the second tube portion (5) being electrically connected,
further **characterized in that** either
the first connecting element (9) carries in the region of the periphery of its flange (12) an electrical reading unit (19), and the second connecting element (10) carries in the region of the periphery of its flange (14) a machine-readable identity element (20), the identity element (20) and the reading unit (21) immediately facing each other in the final position of the two flanges (12, 14),
or
a gas supply (23), to which the tube set is connected, carries an electrical reading unit (19), and the second connecting element (10) carries in the region of the periphery of its flange (14) a machine-readable identity element (20) in the form of a transponder.

2. The tube set according to claim 1, wherein the second connecting element (10) carries the sterile filter element (11).

3. The tube set according to claim 1 or 2, wherein one of the connecting elements (9, 10) is formed by a flange (12) with an internal thread (13), and the other connecting element (9, 10) is formed by a flange (14) with an external thread (15) being complementary to the internal thread (13), and wherein the flanges (12, 14) are connected with each other in a gas-tight manner by bolting into a final position.

4. The tube set according to claim 1 or 2, wherein one of the connecting elements (9, 10) is formed by a flange (12) with spaced latching projections (16) protruding radially inwards, and the other connecting element (9, 10) is formed by a flange (14) with radially extending retaining projections (17), wherein the retaining projections (17), in relation to the circumferential direction of the flange (14), have a distance that is greater than the length of the latching projections (16), and wherein the retaining projections (17) and the latching projections (16) can be brought into engagement with each other by rotating the two flanges (12, 14) against each other into a final position and connect the flanges (12, 14) with each other in a gas-tight manner.

5. The tube set according to claim 1 or 2, wherein one of the connecting elements (9, 10) is formed by a flange (14) with at least one latching recess (17) arranged in the region of the periphery of the flange (14), and the other connecting element (9, 10) is formed by a flange (12) with at least one latching element (18) arranged in the region of the periphery, wherein the latching recess (17) and the latching element (18) are complementary to each other and connect the flanges (12, 14) with each other by latching into each other into a final position in a gas-tight manner.

6. The tube set according to claim 2 or 3,
wherein one of the connecting elements (9, 10) comprises a flange sleeve, the inner diameter of which is in the opening region greater than the outer diameter of the flange (14) of the other connecting element (9, 10), and wherein the inner diameter of the flange sleeve, in relation to the direction of insertion of the flange (14) of the other connecting element (9, 10), decreases to an inner diameter, which is smaller than the outer diameter of the flange (14) of the other connecting element (9, 10), wherein at least one of the components, flange sleeve and/or flange (14) is made of a plastic or elastic material, or
wherein one of the connecting elements (9, 10) comprises a flange sleeve with a cylindrical inner wall, wherein the outer diameter of the flange (14), in relation to the direction of insertion of the flange (14) of the other connecting element (9, 10), decreases from an outer diameter, which is greater than the inner diameter of the flange sleeve, to an outer diameter, which is smaller than the inner diameter the flange sleeve, wherein at least one of the components, flange sleeve and/or flange (14) is made of a plastic or elastic material.

7. The tube set according to one of claims 1 to 6, wherein the first connecting element (9) carries in the region of the periphery of its flange (12) a first electrical connection (21), and the second connecting element (10) carries in the region of the periphery of its flange (14) a second electrical connection (22), with the second connection (22) a humidity sensor arranged in the second tube portion (5) being electrically connected.

8. The tube set according to claim 7, wherein with the first electrical connection (21) a wire connection for the connection to the device (1) for insufflating and/or a transmitter for the wireless transmission of data to the device (1) for insufflating is electrically connected.

9. The tube set according to claim 1, wherein the identity element (20) comprises a code element being different for every second tube portion (5), and wherein the identity element (20) preferably comprises a security element.

10. A device (1) for insufflating gas into a human or animal body during laparoscopy comprising a gas supply (23) with a gas connection (24), to which a tube set according to one of claims 1 to 10 is connected by means of the first connection (3), and comprising an insufflation instrument (8) connected to the fourth connection (7) and comprising a control circuitry for controlling the heating device of the second tube portion (5).

11. The device according to claim 10, comprising an electrical connection (25), to which the reading unit (19) and/or the first electrical connection (21) of the tube set can detachably be connected, and/or comprising a receiver for receiving data transmitted by means of the transmitter of the tube set.

## Revendications

1. Jeu de tuyaux pour un dispositif (1) pour insuffler un gaz dans un corps humain ou animal pendant la laparoscopie,
avec une première partie de tuyau (2), qui comprend un premier raccord (3) et un deuxième raccord (4), le premier raccord étant connectable au dispositif (1) pour insuffler, et
avec une deuxième partie de tuyau (5), qui comprend un troisième raccord (6) et un quatrième raccord (7), le quatrième raccord (7) étant connectable à un instrument d'insufflation (8),
le deuxième raccord (4) comprenant un premier élément de liaison (9), et le troisième raccord (6) comprenant un deuxième élément de liaison (10), le premier élément de liaison (9) et le deuxième élément de liaison (10) étant configurés mécaniquement complémentaires l'un à l'autre, au moyen des éléments de connexion (9, 10) le deuxième raccord (4) et le troisième raccord (6) étant connectables l'un avec l'autre d'une manière étanche au gaz, et le deuxième ou le troisième raccord (6) comprenant un élément de filtration stérile (11), qui enjambe la coupe transversale intérieure du troisième raccord (6),
**caractérisé en ce que**
le premier élément de liaison (9) porte dans la région de la périphérie de sa bride (12) un premier connecteur électrique (21), et le deuxième élément de liaison (10) porte dans la région de la périphérie de sa bride (14) un deuxième connecteur électrique (22), avec le deuxième connecteur (22) un dispositif de chauffage disposé dans la deuxième partie de tuyau (5) étant électriquement connecté,
en outre **caractérisé en ce qu'**ou
le premier élément de liaison (9) porte dans la région de la périphérie de sa bride (12) une unité de lecture électrique (19), et le deuxième élément de liaison (10) porte dans la région de la périphérie de sa bride (14) un élément d'identité (20) lisible par machine, l'élément d'identité (20) et l'unité de lecture (21) étant immédiatement en regard dans la position finale des deux brides (12, 14),
ou
un appareil d'alimentation en gaz (23), auquel le jeu de tuyaux est lié, porte une unité de lecture électrique (19), et le deuxième élément de liaison (10) porte dans la région de la périphérie de sa bride (14) un élément d'identité (20) en forme d'un transpondeur lisible par machine.

2. Jeu de tuyaux selon la revendication 1, dans lequel le deuxième élément de liaison (10) porte l'élément de filtration stérile (11).

3. Jeu de tuyaux selon la revendication 1 ou 2, dans lequel un des éléments de liaison (9, 10) est formé par une bride (12) avec un filetage intérieur (13), et l'autre élément de liaison (9, 10) est formé par une bride (14) avec un filetage extérieur (15) étant complémentaire au filetage intérieur (13), et dans lequel les brides (12, 14) sont liées l'une avec l'autre d'une manière étanche au gaz par vissage dans une position finale.

4. Jeu de tuyaux selon la revendication 1 ou 2, dans lequel un des éléments de liaison (9, 10) est formé par une bride (12) avec des saillies d'enclenchement (16) espacées et radialement en saillie vers l'intérieur, et l'autre élément de liaison (9, 10) est formé par une bride (14) avec des saillies de retenue (17) s'étendant radialement, dans lequel les saillies de retenue (17), en relation à la direction périphérique de la bride (14), ont une distance, qui est supérieure à la longueur des saillies d'enclenchement (16), et dans lequel les saillies de retenue (17) et les saillies d'enclenchement (16) peuvent être mises en prise les unes avec les autres par rotation des deux brides (12, 14) l'une par rapport à l'autre dans une position finale et lient les brides (12, 14) l'une avec l'autre d'une manière étanche au gaz.

5. Jeu de tuyaux selon la revendication 1 ou 2, dans lequel un des éléments de liaison (9, 10) est formé par une bride (14) avec au moins un creux d'enclenchement (17) disposé dans la région de la périphérie de la bride (14), et l'autre élément de liaison (9, 10) est formé par une bride (12) avec au moins un élément d'enclenchement (18) disposé dans la région de la périphérie, dans lequel le creux d'enclenchement (17) et l'élément d'enclenchement (18) sont complémentaires l'un à l'autre et lient par enclenchement l'un dans l'autre dans une position finale les brides (12, 14) l'un avec l'autre d'une manière étanche au gaz.

6. Jeu de tuyaux selon la revendication 2 ou 3, dans lequel un des éléments de liaison (9, 10) comprend une douille à bride, le diamètre intérieur de laquelle est dans la région d'ouverture supérieur au diamètre extérieur de la bride (14) de l'autre élément de liaison (9, 10), et dans lequel le diamètre intérieur de la douille à bride, en relation à la direction d'insertion de la bride (14) de l'autre élément de liaison (9, 10), décroît à un diamètre intérieur, qui est inférieur au diamètre extérieur de la bride (14) de l'autre élément de liaison (9, 10), dans lequel au moins un des composants, douille à bride et/ou bride (14) est en une matière plastique ou élastique, ou dans lequel un des éléments de liaison (9, 10) comprend une douille à bride avec une paroi intérieure cylindrique, dans lequel le diamètre extérieur de la bride (14), en relation à la direction d'insertion de la bride (14) de l'autre élément de liaison (9, 10), décroît à partir d'un diamètre extérieur, qui est supérieur au diamètre intérieur de la douille à bride, à un diamètre extérieur, qui est inférieur au diamètre intérieur da la douille à bride, dans lequel au moins un des composants, douille à bride et/ou bride (14) est en une matière plastique ou élastique.

7. Jeu de tuyaux selon une des revendications 1 à 6, dans lequel le premier élément de liaison (9) porte dans la région de la périphérie de sa bride (12) un premier connecteur électrique (21), et le deuxième élément de liaison (10) porte dans la région de la périphérie de sa bride (14) un deuxième connecteur électrique (22), avec le deuxième connecteur (22) un capteur d'humidité disposé dans la deuxième partie de tuyau (5) étant électriquement connecté.

8. Jeu de tuyaux selon la revendication 7, dans lequel avec le premier connecteur électrique (21) une connexion à fil pour le raccordement au dispositif (1) pour insuffler et/ou un transmetteur pour la transmission sans fils de données au dispositif (1) pour insuffler est électriquement connectée.

9. Jeu de tuyaux selon la revendication 1, dans lequel l'élément d'identité (20) comprend un élément de codage étant différent pour chaque deuxième partie de tuyau (5), et dans lequel l'élément d'identité (20) de préférence comprend un élément de sécurité.

10. Dispositif (1) pour insuffler un gaz dans un corps humain ou animal pendant la laparoscopie comprenant un appareil d'alimentation en gaz (23) avec un raccord de gaz (24), auquel un jeu de tuyaux selon une des revendications 1 à 10 est lié au moyen du premier raccord (3), et comprenant un instrument d'insufflation (8) lié au quatrième raccord (7) et comprenant un circuit de commande pour commander le dispositif de chauffage de la deuxième partie de tuyau (5).

11. Dispositif selon la revendication 10, comprenant un connecteur électrique (25), auquel l'unité de lecture (19) et/ou le premier connecteur électrique (21) du jeu de tuyaux peu être lié de manière dissociable, et/ou comprenant un récepteur pour recevoir des données transmises au moyen du transmetteur du jeu de tuyaux.
